# EUROPEAN PATENT APPLICATION

(11) **EP 2 123 773 A1**
(43) Date of publication of application: **25.11.2009**
(21) Application number: 08009331.3
(22) Date of filing: 21.05.2008
(51) Int. Cl.: C12Q 1/68

(54) **Use of GPR151 modulators for the treatment of pain**

(71) Applicant: sanofi-aventis, 75013 Paris (FR)
(72) Inventor: The designation of the inventor has not yet been filed
(74) Representative: Schneider, Rudolf

(57) **Abstract**

The present invention relates to nucleic acid arrays used to detect genes which are up or down regulated following a pain stimulus in a mammal. It further relates to the use of GPR151 in methods to detect pain or evaluate the effect of a compound on the level of pain in a mammal. Other aspects of the invention concern a method for screening pharmaceuticals, methods for the treatment of pain and a pharmaceutical composition comprising a GPR151 modulator for the treatment of pain.

## Description

The present invention relates to nucleic acid arrays used to detect genes which are up or down regulated following a pain stimulus in a mammal. It further relates to the use of GPR151 in methods to detect pain or evaluate the effect of a compound on the level of pain in a mammal. Other aspects of the invention concern a method for screening pharmaceuticals, methods for the treatment of pain and a pharmaceutical composition comprising a GPR151 modulator for the treatment of pain.

Pain is a complex subjective sensation reflecting real or potential tissue damage and the affective response to it. Acute pain is a physiological signal indicating a potential or actual injury. Chronic pain can either be somatogenetic (organic) or psychogenic. Chronic pain is frequently accompanied or followed by vegetative signs, which often result in depression.

Somatogenetic pain may be of nociceptive origin, inflammatory or neuropathic. Nociceptive pain is judged to be commensurate with ongoing activation of somatic or visceral pain-sensitive nerve fibers. Neuropathic pain results from dysfunction in the nervous system; it is believed to be sustained by aberrant somatosensory processes in the peripheral nervous system, the CNS, or both. (For an overview of pain mechanisms, see for example Scholz and Woolf, 2002; Julius and Basbaum, 2001, Woolf and Mannion, 1999; Wood, J.D., 2000; Woolf and Salter, 2000.)

Chronic pain results in individual suffering and social economic costs of tremendous extent. Existing pharmacological pain therapies are widely unsatisfying both in terms of efficacy and of safety.

Up to now, two classes of analgesics are mainly employed for the treatment of pain: Non-opioid analgesics, mostly acetaminophen and NSAIDS (non-steroidal anti-inflammatory drugs) and opioid (narcotic) agonists (wherein "opioid" is a generic term for natural or synthetic substances that bind to specific opioid receptors in the CNS, producing an agonist action). Unfortunately both analgesic classes, opioids and non-opioids, have several unwanted side effects. The most serious side effects of opioids are the possibility of inhibition of the respiratory system and after long-term treatment the possibility of addiction (Schaible H.G., Vanegas H., 2000). NSAIDs, a major class of non-opioids, on the other hand, can induce a variety of gastrointestinal complications such as ulcers and bleeding, but also kidney damage (Schaible H.G., Vanegas H., 2000). It has been estimated that in the U.S.A. about 16.000 patients die every year because of severe gastro-intestinal complications caused by conventional NSAIDs.

In light of the severe drawbacks connected with state of the art pain treatments, there is a great need for novel classes of pain modulating drugs. Especially in light of the vast gap between the fast advancing understanding of the neurobiology of pain and the unmet clinical need to provide effective treatments without the drawbacks of state of the art treatments, efforts need to be directed to the discovery of new targets for novel classes of analgesics.

Thus, it is the object of the present invention to provide a new means for the development and provision of a new class of pain modulating drugs.

This object is solved by the use of GPR151 modulators for the preparation of pharmaceutical compounds that modulate pain.

The invention is based on findings of the inventors that demonstrate for the first time the surprising implication of GPR151 in the forwarding and the processing of nociceptive signals.

One subject matter of the present invention is directed to an array comprising polynucleotide probes capable of hybridizing to nucleic acid molecules of one or more genes coding for proteins involved in the perception, the forwarding, the processing of pain stimuli, and/or the triggering of operating reactions to disengage from the pain stimulus.
Said polynucleotide probes are stably associated with a support made from glass or nylon and hybridize to nucleic acid molecules derived from a cell of a subject suffering of, or suspected to suffer of, pain. The polynucleotide probes may be PCR products, long oligonucleotides or short oligonucleotides synthesized in situ. Said nucleic acid molecules may be RNA or cDNA.

In a more specific matter, the array comprises polynucleotide probes capable of hybridizing to nucleic acid molecules of one or more genes coding for proteins involved in processes occurring in a cell following pain stimuli. In particular, said cell can be a neuronal cell coming from a mammal, such as a mouse, a rat, a dog, a bovine, a pig, an ape or a human.
In a further more specific matter, said proteins are receptors, channels, transport proteins, signaling factors, phosphatases, kinases or nucleic hormones.
In a preferred embodiment of the present invention, said protein is a G protein-coupled receptor 151 (GPR151).

In a preferred embodiment, the array according to the present invention is used for detecting changes in gene expression upon pain stimulation or for detecting changes in gene expression in a subject that is not capable of expressing that it is suffering of pain, e.g. an animal, a child, a senile person or a mentally disabled person.

For the different aspects of present invention, pain may be acute pain or chronic pain; neuropathic pain, head pain, throat pain, tooth pain, ear pain, eye pain, neck pain, back pain, breast pain, abdominal pain, limb pain, joint pain, cutaneous pain, somatic pain, visceral pain or phantom limb pain; pain my be caused by an injury, an infection, an inflammation, a cancer or a disease; or by a thermal stimulus, a mechanical stimulus, an electric stimulus a chemical stimulus or a radioactive stimulus.

Another subject of the present invention is directed to an antisense oligonucleotide molecule, a ribozyme molecule, an interfering RNA molecule, an antibody or antibody fragment that can specifically inhibit expression of a mammalian GPR151 gene, and to the use of said antibody or antibody fragment for inhibiting the expression of a mammalian GPR151 gene or for diagnosing that a subject is suffering of pain.

The invention further provides a cell which has been genetically modified so that the normal expression of a GPR151 encoding gene has been reduced or eliminated; a genetically modified mammal that comprises such modified cells, said mammal being a mouse, a rat, a dog, a bovine, a pig, an ape or a human; and a method of producing such a genetically modified mammal, comprising genetically modifying one or more cells of said mammal such that the expression of the GPR151 gene in said mammal has been reduced or eliminated as compared to the expression of the GPR151 gene in a wild-type mammal.

A preferred subject of the present invention refers to a method of diagnosing a subject suffering of pain, comprising determining the expression level of the GPR151 protein in a biological sample from said subject. Said method can comprise the steps:
a) obtaining a tissue sample from a subject;
b) isolating RNA from the sample;
c) incubating the RNA with the array of any one of claims 1 to 17; and
d) analyzing the gene expression on the array.

Another preferred subject refers to a method of screening for a compound capable of modulating GPR151 gene expression in a cell, comprising:
a) contacting a cell with a test compound;
b) preparing a RNA sample from said cell; and
c) incubating the RNA sample to the nucleic acid array according to the invention,
wherein a change in GPR151 gene expression profile in said cell after said contacting, as compared to before said contacting, is indicative that said compound is capable of modulating GPR151 gene expression in said cell.

In a preferred embodiment, the invention provides a method to treat or ameliorate pain, comprising administering to a subject in need thereof an effective amount of a GPR151 modulator. Said method can comprise the steps:
a) determining the expression level of the GPR151 protein in a subject; and
b) administering a GPR151 modulator to a subject having an increased expression level of the GPR151 protein as compared to a control.
   Step a) may be carried out by using the nucleic acid array according to the invention. The GPR151 modulator used in said method is selected from the group consisting of antisense oligonucleotides, ribozymes, double stranded RNA, antibodies or antibody fragments, wherein said molecules are designed to inhibit GPR151 gene expression.

In another preferred embodiment, the invention provides a method of evaluating the effect of a compound on the level of pain in a mammal, comprising:
a) administering the compound to the mammal having pain;
b) obtaining at least one cell sample from the mammal; and
c) determining the expression level of the GPR151 protein in the cell sample;
wherein a difference between the expression level of the GPR151 protein determined in step c) and the expression level of the GPR151 protein in the absence of the compound indicates the level of efficacy of the compound on pain.
Step c) may be carried out by using the nucleic acid array according to the invention.

Moreover, a pharmaceutical composition comprising a GPR151 modulator in an effective amount to treat or ameliorate pain in a subject in need thereof is encompassed by the present invention. Said modulator is selected from the group consisting of antisense oligonucleotides, ribozymes, double stranded RNA, antibodies or antibody fragments, wherein said molecules are designed to inhibit GPR151 gene expression. In a preferred embodiment, the a pharmaceutical composition comprising a GPR151 modulator is used to treat or ameliorate neuropathic pain.

Pain is defined by the International Association for the Study of Pain (IASP) as "an unpleasant sensory and emotional experience associated with actual or potential tissue damage, or described in terms of such damage".

Pain is the most common symptom for which patients seek medical help, and can be classified as either "acute" or "chronic".

"Acute pain" is precipitated by immediate tissue injury (e. g., a burn or a cut), and is usually self-limited. This form of pain is a natural defense mechanism in response to immediate tissue injury, preventing further use of the injured body part, and withdrawal from the painful stimulus. It is amenable to traditional pain therapeutics, including non-steroidal anti-inflammatory drugs (NSAIDs) and opioids.

In contrast, "chronic pain" is present for an extended period, e. g., for three or more months, persisting after an injury has resolved. Chronic pain may have no apparent cause or may be caused by a developing illness or imbalance. This disorder can trigger multiple psychological problems that confound both patient and health care provider. Sometimes chronic pain can have a psychosomatic or psychogenic cause.

Pain can further be classified as either "nociceptive" or "neuropathic", as defined below.

"Nociceptive pain" is due to activation of pain-sensitive nerve fibers, either somatic or visceral. Nociceptive pain is generally a response to direct tissue damage. The initial trauma typically causes the release of several chemicals including bradykinin, serotonin, substance P, histamine, and prostaglandin. When somatic nerves are involved, the pain is typically experienced as an aching or pressure-like sensation.

Nociceptive pain has traditionally been managed by administering non-opioid analgesics. These analgesics include acetylsalicylic acid, choline magnesium trisalicylate, acetaminophen, ibuprofen, fenoprofen, diflusinal, and naproxen, among others. Opioid analgesics, such as morphine, hydromorphone, methadone, levorphanol, fentanyl, oxycodone and oxymorphone, may also be used.

The term "neuropathic pain" refers to pain that is due to injury or disease of the central or peripheral nervous system (McQuay, Acta Anaesthesiol. Scand. 1997; 41(1 Pt 2): 175-83; Portenoy, J. Clin. Oncol. 1992; 10:1830-2). In contrast to the immediate pain caused by tissue injury, neuropathic pain can develop days or months after a traumatic injury. Furthermore, while pain caused by tissue injury is usually limited in duration to the period of tissue repair, neuropathic pain frequently is long-lasting or chronic. Moreover, neuropathic pain can occur spontaneously or as a result of stimulation that normally is not painful. Neuropathic pain is associated with chronic sensory disturbances, including spontaneous pain, hyperalgesia (i. e., sensation of more pain than the stimulus would warrant), and allodynia (i. e., a condition in which ordinarily painless stimuli induce the experience of pain). In humans, prevalent symptoms include cold hyperalgesia and mechanical allodynia. Sensitivity to heat is rarely reported. Descriptors that are often used to describe such pain include "lancinating", "burning", or "electric". Examples of neuropathic pain syndromes include those due to disease progression, such as diabetic neuropathy, multiple sclerosis, or post-herpetic neuralgia (shingles) ; those initiated by injury, such as amputation (phantom-limb pain), or injuries sustained in an accident (e. g., avulsions) ; and those caused by nerve damage, such as from chronic alcoholism, viral infection, hypothyroidism, uremia, or vitamin deficiencies. Traumatic nerve injuries can also cause the formation of neuromas, in which pain occurs as a result of aberrant nerve regeneration. Stroke (spinal or brain) and spinal cord injury can also induce neuropathic pain. Cancer-related neuropathic pain results from tumor growth compression of adjacent nerves, brain, or spinal cord. In addition, cancer treatments, including chemotherapy and radiation therapy, can also cause nerve injury.

These persistent neuropathic pains, which do not respond well to traditional aspirinlike or opioid drugs, are thought to arise from the development of long-term changes in the processing of pain messages by spinal cord and brainstem cells. Many of these changes are associated with the induction of genes, which genes represent interesting molecular targets that need to be studied and that may be used in screening methods in order to identify new analgesic compounds.

The present invention provides means to study such molecular targets, in particular a nucleic acid array comprising polynucleotide probes capable of hybridizing to nucleic acid molecules of one or more genes coding for proteins involved in the perception, the forwarding, the processing of pain stimuli, and/or the triggering of operating reactions to disengage from the pain stimulus.

As used herein, "array" refers to an orderly arrangement of probes that provides a medium for matching known and unknown DNA samples and automated process of identifying the unknowns.
It includes arrays known in the art, such as a DNA chip, a DNA microarray and a DNA macroarray, and the like, and comprises a solid support and plural polynucleotides or fragments thereof which are adhered to the surface of the solid support.
Examples of the solid support include a glass plate, a nylon membrane, and the like.

The polynucleotides or fragments thereof adhered to the surface of the solid support can be adhered to the surface of the solid support using the general technique for preparing arrays. Namely, a method in which they are adhered to a chemically surface-treated solid support, for example, to which a polycation such as polylysine or the like has been adhered (Nat. Genet., 21: 15-19 (1999)). The chemically surface-treated supports are commercially available and the commercially available solid product can be used, as the solid support of the polynucleotide array according to the present invention.

The arrays according to the present invention are preferably nucleic acid arrays that comprise a plurality of polynucleotide probes immobilized on a surface or substrate. The different polynucleotide probes are complementary to, and therefore can hybridize to, different target nucleic acid molecules in a sample. Thus, such probes can be used to simultaneously detect the presence and quantity of a plurality of different nucleic acid molecules in a sample, to determine the expression level of a plurality of different genes, e. g., the presence and abundance of different mRNA molecules, or of nucleic acid molecules derived therefrom (for example cDNA).

Typically, arrays are described as macroarrays or microarrays. Macroarrays contain sample spot sizes of about 300 microns or larger. The sample sizes in microarray are 300 or less microns but typically less than 200 microns in diameter. Microarrays can utilize specialized robotics and/or imaging equipment to enhance throughput and visualization of data.

Microarrays are useful for simultaneously detecting the presence, absence and quantity of a plurality of different target molecules in a sample (such as an mRNA preparation isolated from a relevant cell, tissue, or organism, or a corresponding cDNA or cRNA preparation). The presence and quantity, or absence, of a probe's target molecule in a sample may be readily determined by analyzing whether (and how much of) a target has bound to a probe at a particular location on the surface or substrate.

As used herein, the term "nucleic acid" or "nucleic acid molecule" refers to a polymeric form of nucleotides of any length, either ribonucleotides or deoxyribonucleotides, in either single- or double-stranded from, and that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. The nucleic acid molecules according to the present invention are preferably mRNA or cDNA.

As used herein, the term "polynucleotide probe" refers to an oligodeoxyribonucleotide or oligoribonucleotide, or any modified form of these polymers that are capable of hybridizing with a target nucleic acid sequence by complementary base-pairing. Complementary base pairing means sequence-specific base pairing that includes e.g., Watson-Crick base pairing as well as other forms of base pairing such as Hoogsteen base pairing. Modified forms include 2'-O-methyl oligoribonucleotides and so-calls PNAs, in which oligodeoxyribonucleotides are linked via peptide bonds rather than phosphodiester bonds.

The term "hybridizing" refers to anti-parallel hydrogen bonding between two single-stranded nucleic acids, in which A pairs with T (or U if an RNA nucleic acid) and C pairs with G. Nucleic acid molecules are "hybridizable" to each other when at least one strand of one nucleic acid molecule can form hydrogen bonds with the complementary bases of another nucleic acid molecule under defined stringency conditions. Stringency of hybridization is determined, e. g., by (i) the temperature at which hybridization and/or washing is performed, and (ii) the ionic strength and (iii) concentration of denaturants such as formamide of the hybridization and washing solutions, as well as other parameters. Hybridization requires that the two strands contain substantially complementary sequences. Depending on the stringency of hybridization, however, some degree of mismatches may be tolerated. See Molecular Biology of the Cell, Alberts et al., 3rd ed., New York and London: Garland Publ., 1994, Ch. 7.

The term "G protein-coupled receptor 151" or "GPR151" in context of the present invention refers to a member of the GPR family. It is already known in the literature with the NCBI polynucleotide sequence reference NM_194251.
GPR151 could be derived from any vertebrate and in particular mammalian species (e.g. dog, horse, bovine, mouse, rat, canine, rabbit, chicken, anthropoid, human or others). GPR151 could be isolated from tissue probes of such vertebrate organisms or could be manufactured by means of recombinant biological material that is able to express the GPR151 protein.
The term may refer to native polypeptides, polymorphic variants, mutants, and interspecies homologues.

Preferred neuronal cells according to the present invention are dorsal root ganglion cells, spinal chord cells and thalamus cells.
Preferred cells used in the methods according to the invention are MDCK, HEK 293, HEK 293 T, BHK, COS, NIH3T3, Swiss3T3 and CHO. The cell will usually be a mammalian cell, such as a human cell, a mouse cell, a rat cell, a Chinese hamster cell, etc.

An "antisense" oligonucleotide molecule is a single stranded nucleic acid molecule, which may be DNA, RNA, a DNA-RNA chimera, or a derivative thereof, which, upon hybridizing under physiological conditions with complementary bases in an RNA or DNA molecule of interest, inhibits the expression of the corresponding gene by inhibiting, e. g., mRNA transcription, mRNA splicing, mRNA transport, or mRNA translation or by decreasing mRNA stability. As presently used, "antisense" broadly includes RNA-RNA interactions, RNA-DNA interactions, and RNase-H mediated arrest. Antisense nucleic acid molecules can be encoded by a recombinant gene for expression in a cell (see, e.g., U. S. Patents No. 5,814,500 and 5,811,234), or alternatively they can be prepared synthetically (see, e. g., U. S. Patent No. 5,780,607).

The term "ribozyme" is used to refer to a catalytic RNA molecule capable of cleaving RNA substrates. Ribozyme specificity is dependent on complementary RNA-RNA interactions (for a review, see Cech and Bass,Annu. Rev. Biochem. 1986; 55: 599-629). Two types of ribozymes, hammerhead and hairpin, have been described. Each has a structurally distinct catalytic center. Ribozyme technology is described further in Intracellular Ribozyme Applications: Principals and Protocols, Rossi and Couture ed., Horizon Scientific Press, 1999.

The term "interfering RNA" refers to the ability of double stranded RNA (dsRNA) to suppress the expression of a specific gene of interest in a homology-dependent manner. It is currently believed that RNA interference acts post-transcriptionally by targeting mRNA molecules for degradation. RNA interference commonly involves the use of dsRNAs that are greater than 500 bp; however, it can also be mediated through small interfering RNAs (siRNAs) or small hairpin RNAs (shRNAs), which can be 10 or more nucleotides in length and are typically 18 or more nucleotides in length. For reviews, see Bosner and Labouesse, Nature Cell Biol. 2000; 2: E31-E36 and Sharp and Zamore, Science 2000; 287: 2431-2433.

The term "antibody" is used herein in the broadest sense and specifically covers monoclonal antibodies (including full length monoclonal antibodies) of any isotype such as IgG, IgM, IgA, IgD, and IgE, polyclonal antibodies, multispecific antibodies, chimeric antibodies, and antibody fragments. An antibody reactive with a specific antigen can be generated by recombinant methods such as selection of libraries of recombinant antibodies in phage or similar vectors, or by immunizing an animal with the antigen or an antigen-encoding nucleic acid.

The term "subject" as used herein refers to a mammal (e. g., a rodent such as a mouse or a rat, a pig, a primate, a dog or a cat, etc.) In particular, the term refers to humans.

As used herein, the term "genetically modified animal" encompasses all animals besides humans into which an exogenous genetic material has been introduced and/or whose endogenous genetic material has been manipulated. Examples of genetically modified animals include without limitation transgenic animals, e. g. , "knock-in" animals with the endogenous gene substituted with a heterologous gene or an ortholog from another species or a mutated gene, "knockout" animals with the endogenous gene partially or completely inactivated, or transgenic animals expressing a mutated gene or overexpressing a wild-type or mutated gene (e.g., upon targeted or random integration into the genome) and animals containing cells harboring a non-integrated nucleic acid construct (e. g. , viral-based vector, antisense oligonucleotide, shRNA, siRNA, ribozyme, etc. ), including animals wherein the expression of an endogenous gene has been modulated (e. g. , increased or decreased) due to the presence of such construct.

For an overview of the above techniques, see for example: Current protocols in Molecular biology (2000) J.G. Seidman, Chapter 23, Supplemtent 52, John Wiley and Sons, Inc.; Gene Targeting: a practical approach (1995), Editor: A.L. Joyner, IRL Press; Genetic Manipulation of Receptor Expression and Function, 2000; Antisense Therapeutics, 1996; Scherr et al, 2003.

The term "GPR151 modulator" means a modulating molecule of the GPR151 receptor, in particular an inhibitory or activating molecule ("inhibitor" or "activator"), especially an inhibitor of the GPR151 receptor identifiable according to the method of the present invention.
An inhibitor is generally a compound which, e.g. binds to, partially or totally blocks activity, decreases, prevents, delays activation, inactivates, desensitizes, or down-regulates the activity or expression of the GPR151 receptor. An activator is generally a compound which, e.g. increases, opens, activates, facilitates, enhances activation, sensitizes, agonizes, or up-regulates the activity or expression of the GPR151 receptor.
Such modulators include naturally occurring or synthetic ligands, antagonists, agonists, peptides, cyclic peptides, nucleic acids, antibodies, antisense molecules, ribozymes, small organic molecules and the like.

A "test compound" is a molecule that can be tested for its ability to act as a modulator of a gene or gene product. Test compounds can be selected without limitation from small inorganic and organic molecules(i. e. , those molecules of less than about 2 kD, and more preferably less than about 1 kD in molecular weight), polypeptides (including native ligands, antibodies, antibody fragments, and other immunospecific molecules), oligonucleotides, polynucleotide molecules, and derivatives thereof.

In the context of the present invention, the term "treat" refers to a means to relieve or alleviate the perception of a pain. It may mean to relieve or alleviate the intensity and/or duration of a pain (e. g., burning sensation, tingling, electric-shock-like feelings, etc.) experienced by a subject in response to a given stimulus (e. g., pressure, tissue injury, cold temperature, etc.). Treatment can occur in a subject (e. g., a human or companion animal) suffering from a pain condition or having one or more symptoms of another condition that can be treated according to the present invention, or in an animal model of pain, such as the SNI (spared nerve injury) rat model of neuropathic pain described herein, or any other animal model of pain.

The term "pharmaceutical composition" refers to a composition comprising a therapeutically effective amount of a GPR151 modulator such as described above and one or more pharmaceutically acceptable carriers or auxiliary substances.
The pharmaceutical composition can be administered systemically or topically, in any conventional manner. This can e.g. be by means of oral dosage forms, such as, for example, tablets or capsules, by means of the mucous membranes, for example the nose or the oral cavity, in the form of dispositories implanted under the skin, by means of injections, infusions or gels which contain the medicaments according to the invention. It is further possible to administer the pharmaceutical composition topically and locally in order to treat the particular disease as described above, if appropriate, in the form of liposome complexes.

The pharmaceutical composition can also be administered in form of an injection solution or an infusion. Injection solutions are in general used if only relatively small amounts of a solution or suspension, for example about 1 to about 20 ml, are to be administered to the body. Infusion solutions are in general used if a larger amount of a solution or suspension, for example one or more litres, are to be administered. In the case of the administration of relatively large amounts, however, the formulation according to the invention should be diluted briefly before administration to such an extent that an at least approximately isotonic solution is obtained. An example of an isotonic solution is a 0.9% strength sodium chloride solution. In the case of infusion, the dilution can be carried out, for example, using sterile water while the administration can be carried out, for example, via a so-called bypass.

The following Figures and Examples shall explain the present invention without limiting the scope of the invention.

### Examples

### Total RNA Isolation:

Total RNA from DRGs was isolated with the PicoPure RNA Isolation Kit (Arcturus) following the manufacturer's instructions. RNA quality was assessed using the 2100 Bioanalyzer and RNA 6000 Nano LabChip kit (Agilent).

### Affymetrix GeneChip^{™} Microarrays:

First-strand cDNA synthesis was performed using 500ng total RNA with a 100pM T7-(dT)24 oligomer (GGCCAGTGAATTGTAATACGACTCACTATAGGGAGGCGG-dT₂₄) according to Baugh, L.R, Hill, A.A., Brown, E.L. and Hunter, C.P. (2001) Nucleic Acids Res. 29, e29 and SuperScript II Reverse Transcriptase following the manufacturer's instructions. Double-stranded cDNA was synthesized and then extracted using phenol-chloroform followed by an ethanol precipitation step. An *in vitro* transcription reaction was performed with the doublestranded cDNA sample using the BioArray High Yield RNA Transcription Labeling kit (Enzo) according to the manufacturer's instructions. Transcription reactions were incubated at 37°C for 16h. cRNA was purified using the RNeasy Mini kit (Qiagen) protocol for RNA cleanup and quantified by a spectrophotometer. The biotin-labeled cRNA was fragmented using a RNA fragmentation buffer (200mM Tris-acetate, 500mM KOAc, 150mM MgOAc, pH 8.1). Hybridization and staining of mouse MG430 2.0 GeneChips^{™} (Affymetrix) was performed according to the manufacturer's instructions. The microarrays were scanned using a GeneChip 3000 Scanner, and the scanned data were imported and analyzed using Resolver v5.1 expression data analysis software (Rosetta Biosoftware).

### Surgical nerve lesion and sham surgical procedures:

In anesthetized mice, the left sciatic nerve was exposed at its trifurcation and the two major branches (tibial and common peroneal nerves) were ligated and transected distal to the ligation. Sham surgery was identical, the nerves were exposed but not ligated and transected (see Bourquin et al. 2006).

### Determination of paw withdrawal threshold:

Paw withdrawal thresholds (PWTs) were assessed using a dynamic plantar aesthesiometer (see Szabo et al. 2005). After acclimation in a compartment with metal mesh floor, the stimulator was positioned under the animal's hindpaw, a straight metal filament driven by an electrodynamic actuator touched the plantar surface and exerted an increasing upward force until the animal removed the paw (paw withdrawal threshold, PWT). PWTs were assessed for hindpaws of the ipsilateral, operated side and of the contralateral side.

### TaqMan analysis:

cDNA synthesis was performed using the Applera Ltd. (Norwalk, CT, USA) Reverse Transcriptase Kit (N8080234) and RNase Inhibitor (N8080119). cDNA synthesis was performed from 90ng human RNA samples using 2.2µL 10XRT buffer, 4.84 µL MgCl2 (25mM), 4.4 µL dNTPs (10mM), 1.1 µL random hexamers (50µM), 1.1 µL oligo(dT)16 (50µM), 0.44 µL RNase Inhibitor (20U/µL), 0.55µL Multiscribe (50U/µL) in a total volume of 22 µL (final cDNA concentration: 4 ng/µl). Samples were incubated at 25°C for 10 minutes and 42°C for 60 minutes. The reaction was stopped by heating to 95°C for 5 minutes.
TaqMan reactions were performed using Applera TaqMan Universal PCR Master Mix (4305719) and TaqMan Human GAPDH Control Reagents. Human GPR151-specific oligonucleotides were synthesized by Operon Biotechnologies GmbH (Köln, Germany). PCR was performed using the ABI Prism 7900 (Applera) under the following PCR conditions: 2 minutes at 50°C, 10 minutes at 95°C, 40 cycles with 95°C for 15 s and 1 minute at 60°C. PCR was set up as a multiplex PCR using 0.125 µL target probe (50µM), 0.45 µL target forward primer (50µM), 0.45µL target reverse primer (50µM), 12.5 µL TaqMan 2x PCR Master Mix, 0.25µL each of primers and probes (TaqMan Human GAPDH Control Reagents), 2.5 µL or 1.25 µl cDNA sample in a total reaction volume of 25µl.

### Reference:

Bourquin AF et al (2006) Assessment and analysis of mechanical allodynia-like behavior induced by spared nerve injury (SNI) in the mouse. Pain 122:e1-14. Epub 2006

### Results:

Strong induction of GPR151 mRNA expression in mice by neuropathic pain

Neuropathic pain was induced in mice of strain C57/B6 by SNI (spared nerve injury) operation and phenotypically checked two days and four days after SNI operation by quantification of paw withdrawal thresholds. Four days after SNI operation animals were sacrificed and the spinal cord as well as L4 and L5 dorsal root ganglia from single animals were prepared. After pooling of L4 and L5 dorsal root ganglia from particular animals, total RNA was extracted from said pooled dorsal root ganglia or from spinal cord samples. 500 ng RNA was amplified and hybridized on MG430 2.0 GeneChip^{™} microarrays from Affymetrix representing the whole genome of mice. The microarrays were washed, stained and scanned. The gene expression intensities were normalized and analyzed by Resolver software version 5.1.The figures of the table represent the mean expression intensity of 15 animals each. The mean expression level of GPR151 is increased from 12,80 to 1133 in L4/L5 dorsal root ganglia only when neuropathic pain was induced in the respective animals by SNI operation (table 1).

Table 1 shows the strong transcriptional induction of mouse GPR151 in pooled L4/L5 dorsal root ganglia after induction of neuropathic pain by SNI operation (mouse strain C57/B6, probe set 1457555_at representing GPR151 on GeneChip^{™} MG430 2.0).

**Table 1:**

| Tested model | Mean expression intensity of GPR151 mRNA (probe set 1457555_at on GeneChip^{™} MG430 2.0) |
|---|---|
| L4/L5 dorsal root ganglia (n = 15 animals); formalin assay (acute pain) | 18,20 (+/- 8,01) |
| Spinal cord (n = 15 animals); formalin assay (acute pain) | 3,08 (+/- 7,28) |
| L4/L5 dorsal root ganglia (n = 15 animals); 4 days after SNI operation (neuropathic pain) | 1133,32 (+/- 152,59) |
| Spinal cord (N = 15 animals); 4 days after SNI operation (neuropathic pain) | 7,82 (+/- 6,40) |
| L4/L5 dorsal root ganglia (n = 15 animals); no pain | 12,80 (+/- 7,69) |
| Spinal cord (n = 15 animals); no pain | 7,15 (+/- 10,22) |

### Distribution of GPR151 in human normal tissues

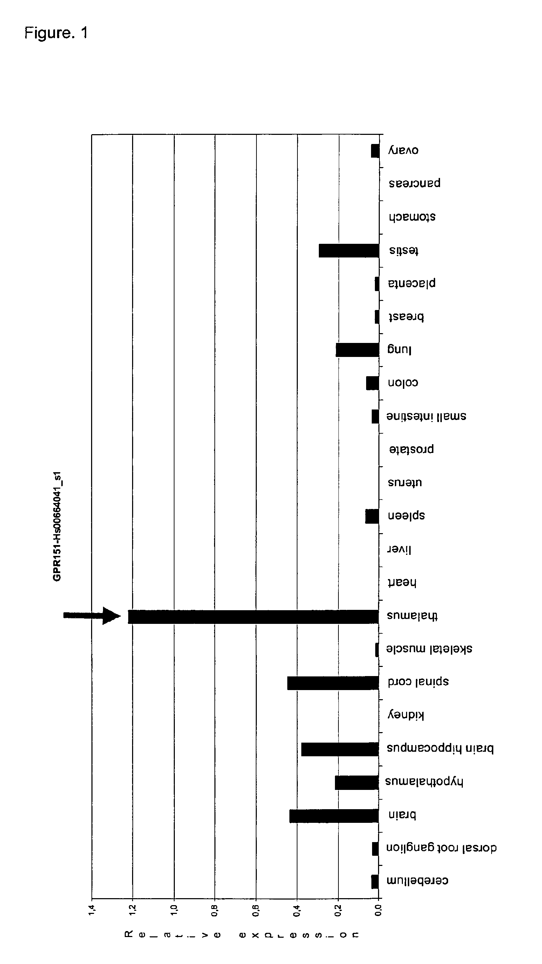

The RNA samples from human normal tissues were analysed for expression of GPR151 mRNA by TaqMan assay using human GPR151 specific primers and probes. The highest level of GPR151 expression is obtained from thalamus (Fig. 1). Thalamus is known that it is relevant for pain transduction and processing in the brain (Herero et al.; Childs Nerv Syst. 18 (2002), 386 - 404). This hints at an important role of GPR151 as a mediator and regulator of pain processing.

Fig. 1. Normal tissue distribution of human GPR151 (TaqMan data)

## Claims

1. An array comprising polynucleotide probes capable of hybridizing to nucleic acid molecules of one or more genes coding for proteins involved in the perception, the forwarding, the processing of pain stimuli, and/or the triggering of operating reactions to disengage from the pain stimulus.

2. An array comprising polynucleotide probes capable of hybridizing to nucleic acid molecules of one or more genes coding for proteins involved in processes occurring in a cell following pain stimuli.

3. The array according to claim 2, wherein said proteins are receptors, channels, transport proteins, signaling factors, phosphatases, kinases or nucleic hormones.

4. The array according to claim 3, wherein one of the polynucleotide probes is capable of hybridizing to a nucleic acid molecule encoding the G protein-coupled receptor 151 (GPR151) gene.

5. The array according to any one of claims 2 to 4, wherein said cell is a neuronal cell.

6. The array according to any one of claims 2 to 5, wherein said cell is from a mammal.

7. The array according to claim 6, wherein said cell is from a mouse, a rat, a dog, a bovine, a pig, an ape or a human.

8. The array according to any one of claims 1 to 7, wherein said polynucleotide probes are PCR products, long oligonucleotides or short oligonucleotides synthesized in situ.

9. The array according to any one of claims 1 to 8, wherein said polynucleotide probes hybridize to nucleic acid molecules derived from a cell of a subject suffering of, or suspected to suffer of, pain.

10. The array according to any one of claims 1 to 9, wherein said nucleic acid molecules are RNA.

11. The array according to any one of claims 1 to 9, wherein said nucleic acid molecules are cDNA.

12. The array according to any one of claims 1 to 11, wherein the polynucleotide probes are stably associated with a support made from glass or nylon.

13. The array according to any one of claims 1 to 12, wherein pain is acute pain or chronic pain.

14. The array according to any one of claims 1 to 13, wherein pain is head pain, throat pain, tooth pain, ear pain, eye pain, neck pain, back pain, breast pain, abdominal pain, limb pain, joint pain, cutaneous pain, somatic pain, visceral pain or phantom limb pain.

15. The array according to any one of claims 1 to 13, wherein pain is neuropathic pain.

16. The array according to any one of claims 1 to 15, wherein pain is caused by an injury, an infection, an inflammation, a cancer or a disease.

17. The array according to any one of claims 1 to 15, wherein pain is caused by a thermal stimulus, a mechanical stimulus, an electric stimulus a chemical stimulus or a radioactive stimulus.

18. Use of the array according to any one of claims 1 to 17, for detecting changes in gene expression upon pain stimulation.

19. Use of the array according to any one of claims 1 to 17, for detecting changes in gene expression in a subject that is not capable of expressing that it is suffering of pain.

20. Use according to claim 19, wherein the subject is an animal, a child, a senile person or a mentally disabled person.

21. An antisense oligonucleotide molecule that can specifically inhibit expression of a mammalian GPR151 gene.

22. A ribozyme molecule that can specifically inhibit expression of a mammalian GPR151 gene.

23. An interfering RNA molecule that can specifically inhibit expression of a mammalian GPR151 gene.

24. An antibody or antibody fragment that can specifically bind to a GPR151 protein.

25. Use of the antibody according to claim 24, for inhibiting the expression of a mammalian GPR151 gene.

26. Use of the antibody according to claim 24, for diagnosing that a subject is suffering of pain.

27. A mammalian cell which has been genetically modified so that the normal expression of a GPR151 encoding gene has been reduced or eliminated.

28. A genetically modified mammal that comprises cells according to claim 27.

29. A method of producing a genetically modified mammal according to claim 28, comprising genetically modifying one or more cells of said mammal such that the expression of the GPR151 gene in said mammal has been reduced or eliminated as compared to the expression of the GPR151 gene in a wild-type mammal.

30. The method according to claim 29, wherein said mammal is a mouse, a rat, a dog, a bovine, a pig, an ape or a human.

31. A method of diagnosing a subject suffering of pain, comprising determining the expression level of the GPR151 protein in a biological sample from said subject.

32. The method according to claim 31, comprising:
a) obtaining a tissue sample from a subject;
b) isolating RNA from the sample;
c) incubating the RNA with the array of any one of claims 1 to 17; and
d) analyzing the gene expression on the array.

33. A method of screening for a compound capable of modulating GPR151 gene expression in a cell, comprising:
a) contacting a cell with a test compound;
b) preparing a RNA sample from said cell; and
c) incubating the RNA sample to the nucleic acid array of any one of claims 1 to 17,
wherein a change in GPR151 gene expression profile in said cell after said contacting, as compared to before said contacting, is indicative that said compound is capable of modulating GPR151 gene expression in said cell.

34. A method to treat or ameliorate pain, comprising administering to a subject in need thereof an effective amount of a GPR151 modulator.

35. The method according to claim 34, comprising:
a) determining the expression level of the GPR151 protein in a subject; and
b) administering a GPR151 modulator to a subject having an increased expression level of the GPR151 protein as compared to a control.

36. The method according to claim 35, wherein step a) is carrying out by using the nucleic acid array of any one of claims 1 to 17.

37. The method according to any one of claims 34 to 36, wherein said modulator is selected from the group consisting of antisense oligonucleotides, ribozymes and double stranded RNA, wherein said molecules are designed to inhibit GPR151 gene expression.

38. The method according to any one of claims 34 to 36, wherein said modulator is an antibody or antibody fragment that can specifically inhibit GPR151 gene expression.

39. A method of evaluating the effect of a compound on the level of pain in a mammal, comprising:
a) administering the compound to the mammal having pain;
b) obtaining at least one cell sample from the mammal; and
c) determining the expression level of the GPR151 protein in the cell sample;
wherein a difference between the expression level of the GPR151 protein determined in step c) and the expression level of the GPR151 protein in the absence of the compound indicates the level of efficacy of the compound on pain.

40. The method according to claim 39, wherein step c) is carrying out by using the nucleic acid array of any one of claims 1 to 17.

41. The method according to any one of claims 31 to 40, wherein pain is acute pain or chronic pain.

42. The method according to any one of claims 31 to 41, wherein pain is head pain, throat pain, tooth pain, ear pain, eye pain, neck pain, back pain, breast pain, abdominal pain, limb pain, joint pain, cutaneous pain, somatic pain, visceral pain or phantom limb pain.

43. The method according to any one of claims 31 to 41, wherein pain is neuropathic pain.

44. The method according to any one of claims 31 to 43, wherein pain is caused by an injury, an infection, an inflammation, a cancer or a disease.

45. The method according to any one of claims 31 to 43, wherein pain is caused by a thermal stimulus, a mechanical stimulus, an electric stimulus, a chemical stimulus or a radioactive stimulus.

46. A pharmaceutical composition comprising a GPR151 modulator in an effective amount to treat or ameliorate pain in a subject in need thereof.

47. The pharmaceutical composition according to claim 46, wherein said modulator is selected from the group consisting of antisense oligonucleotides, ribozymes and double stranded RNA, wherein said substances are designed to inhibit GPR151 gene expression.

48. The pharmaceutical composition according to claim 46, wherein said modulator is an antibody or antibody fragment that can specifically inhibit GPR151 gene expression.

49. The pharmaceutical composition according to any one of claims 46 to 48, wherein pain is acute pain or chronic pain.

50. The pharmaceutical composition according to any one of claims 46 to 49, wherein pain is head pain, throat pain, tooth pain, ear pain, eye pain, neck pain, back pain, breast pain, abdominal pain, limb pain, joint pain, cutaneous pain, somatic pain, visceral pain or phantom limb pain.

51. The pharmaceutical composition according to any one of claims 46 to 49, wherein pain is neuropathic pain.

52. The pharmaceutical composition according to any one of claims 46 to 51, wherein pain is caused by an injury, an infection, an inflammation, a cancer or a disease.

53. The pharmaceutical composition according to any one of claims 46 to 51, wherein pain is caused by a thermal stimulus, a mechanical stimulus, an electric stimulus, a chemical stimulus or a radioactive stimulus.


SEQUENCE LISTING

<110>  Sanofi-Aventis

<120>  Use of GPR151 modulators for the treatment

<130>  DE2008/033

<150>  08009331.3
<151>  2008-05-21

<160>  1

<170>  PatentIn version 3.3

<210>  1
<211>  39
<212>  DNA
<213>  (dt)24 oligomer

<400>  1
ggccagtgaa ttgtaatacg actcactata gggaggcgg                              39